(19) Europäisches Patentamt
European Patent Office
Office européen des brevets

(11) Veröffentlichungsnummer: **0 216 360**
**A2**

(12) # EUROPÄISCHE PATENTANMELDUNG

(21) Anmeldenummer: 86113103.5

(22) Anmeldetag: 24.09.86

(51) Int. Cl.⁴: C 07 D 401/04
C 07 D 405/14, C 07 D 401/14
A 01 N 43/50
//C07D213/80, C07D491/04, C07D213/81, C07D471/14

(30) Priorität: 24.09.85 DE 3534391
17.02.86 DE 3605343

(43) Veröffentlichungstag der Anmeldung: 01.04.87 Patentblatt 87/14

(84) Benannte Vertragsstaaten: AT BE CH DE FR GB IT LI LU NL SE

(71) Anmelder: SCHERING AKTIENGESELLSCHAFT Berlin und Bergkamen
Müllerstrasse 170/178 Postfach 65 03 11
D-1000 Berlin 65(DE)

(72) Erfinder: Nordhoff, Erhard, Dr.
Flughafenstrasse 18
D-1000 Berlin 44(DE)

(72) Erfinder: Franke, Wilfred, Dr.
Karl-Stieler-Strasse 2b
D-1000 Berlin 41(DE)

(72) Erfinder: Arndt, Friedrich, Dr.
Mühlenfeldstrasse 10
D-1000 Berlin 28(DE)

(54) **Imidazolinyl-Derivate, Verfahren zu ihrer Herstellung und diese Verbindungen enthaltende Mittel mit herbizider Wirkung.**

(57) Die Erfindung betrifft neue 2-(2-Imidazolin-2-yl)-pyridin-3-carbonsäurederivate der allgemeinen Formel

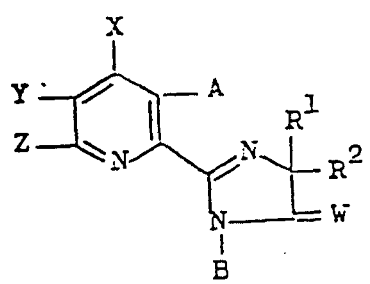

(I)

in der $R^1$, $R^2$, A, B, W, X, Y und Z die in der Beschreibung genannten Bedeutungen haben, Verfahren zu ihrer Herstellung und diese Verbindungen enthaltende Mittel mit herbizider Wirkung.

EP 0 216 360 A2

Croydon Printing Company Ltd.

0216360

## Beschreibung

Die Erfindung betrifft neue Imidazolinyl-Derivate, Verfahren zu ihrer Herstellung und ihre Verwendung als Mittel mit herbizider Wirkung.

Es ist bereits bekannt, daß Imidazoline herbizide Eigenschaften besitzen (DE-OS 28 33 274 und DE-OS 31 21 736). Häufig ist jedoch diese Herbizidwirkung nicht ausreichend, beziehungsweise es treten bei entsprechender Herbizidwirkung Selektivitätsprobleme in landwirtschaftlichen Hauptkulturen auf.

Aufgabe der vorliegenden Erfindung ist die Bereitstellung von neuen Verbindungen, die diese Nachteile nicht aufweisen und in ihren biologischen Eigenschaften den bisher bekannten Verbindungen überlegen sind.

Es wurde gefunden, daß 2-(2-Imidazolin-2-yl)-pyridin-3-carbonsäurederivate der allgemeinen Formel I

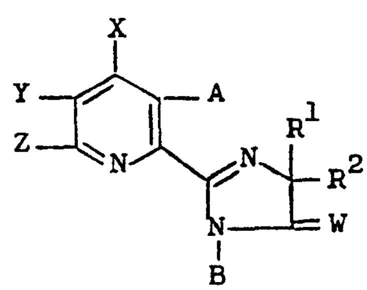

(I) ,

in der

$R^1$ einen $C_1$-$C_4$-Alkylrest,
$R^2$ einen $C_1$-$C_6$-Alkylrest oder einen $C_3$-$C_6$-Cycloalkylrest
oder
$R^1$ und $R^2$ gemeinsam mit dem benachbarten Kohlenstoffatom einen

gegebenenfalls durch Methyl substituierten $C_3$-$C_6$-Cycloalkylrest,

W ein Sauerstoff- oder Schwefelatom,

A eine der Gruppen $CH_2OH$, $COOR^3$ oder $CONR^4R^5$,

$R^3$ ein Wasserstoffatom, einen gegebenenfalls durch ein oder mehrere Sauerstoff- oder Schwefelatome unterbrochenen und gegebenenfalls durch $C_1$-$C_3$-Alkoxy, Halogen, Hydroxy, $C_3$-$C_6$-Cycloalkyl, Benzyl, Furyl, Tetrahydrofuryl, Phenyl, Halogenphenyl, $C_1$-$C_4$-Alkylphenyl, $C_1$-$C_4$-Alkoxyphenyl, Nitrophenyl, Cyano, Carboxyl, $C_1$-$C_4$-Alkoxycarbonyl oder $C_1$-$C_4$-Alkylthio substituierten $C_1$-$C_{12}$-Alkylrest, einen gegebenenfalls durch $C_1$-$C_3$-Alkoxy, Phenyl oder Halogen substituierten $C_3$-$C_8$-Alkenylrest, einen gegebenenfalls durch $C_1$-$C_3$-Alkoxy, Phenyl oder Halogen substituierten $C_3$-$C_8$-Alkinylrest, einen gegebenenfalls durch Methyl substituierten $C_3$-$C_6$-Cycloalkylrest oder ein Kation aus der Gruppe der Alkalimetalle, Erdalkalimetalle, Mangan, Kupfer, Eisen, Zink, Ammonium und organische Ammoniumverbindungen,

$R^4$ und $R^5$ unabhängig voneinander ein Wasserstoffatom, eine Hydroxygruppe oder einen $C_1$-$C_4$-Alkylrest und für den Fall, daß $R^4$ ein Wasserstoffatom ist, $R^5$ auch einen Amino-, Dimethylamino, Acetylamino- oder Anilinorest,

X ein Wasserstoffatom, eine Nitrogruppe, einen $C_1$-$C_3$-Alkylrest, einen $C_1$-$C_3$-Halogenalkylrest oder einen $C_1$-$C_3$-Alkoxyrest und einer der beiden Reste

Y oder Z eine der Gruppen $SR^6$ oder $OR^7$ und der jeweils andere ein Wasserstoffatom, ein Halogenatom, einen $C_1$-$C_4$-Alkylrest, einen $C_1$-$C_4$-Halogenalkylrest, einen $C_1$-$C_4$-Alkoxyrest oder einen $C_1$-$C_4$-Alkylthiorest,

$R^6$ ein Wasserstoffatom, einen Phenylrest, einen Halogenphenylrest, einen $C_1$-$C_3$-Alkylphenylrest, einen Di-$C_1$-$C_3$-alkylphenylrest, einen Nitrophenylrest, einen $C_1$-$C_3$-Alkoxyphenylrest, einen Pyridylrest, einen $C_1$-$C_3$-Alkyl-pyridylrest, einen ein- oder mehrfach durch Sauerstoff unterbrochenen $C_2$-$C_8$-Alkylrest, einen gegebenenfalls durch Sauerstoff, Schwefel, die Imino- oder $C_1$-$C_4$-Alkyliminogruppe unterbrochenen

und gegebenenfalls ein- oder mehrfach durch $C_1$-$C_4$-Alkyl, $C_1$-$C_4$-Alkoxy oder Halogen substituierten $C_3$-$C_8$-Cycloalkylrest, einen $C_5$-$C_8$-Cycloalkenylrest oder einen gegebenenfalls ein- oder mehrfach durch Cyano, Halogen, Hydroxy, Mercapto, Amino, $C_1$-$C_6$-Alkoxy, $C_1$-$C_4$-Alkylthio, Carboxyl, Carbamoyl, $C_1$-$C_4$-Alkoxycarbonyl, $C_1$-$C_4$-Alkylthiocarbonyl, Oxo, Thioxo, Di-$C_1$-$C_3$-alkoxy, Hydroxyimino, $C_3$-$C_8$-Cycloalkyl, $C_3$-$C_8$-Cycloalkenyl, Phenyl, Halogenphenyl, $C_1$-$C_3$-Alkylphenyl, Nitrophenyl, $C_1$-$C_3$-Alkoxyphenyl, Furyl, Tetrahydrofuryl, Dioxolanyl, $C_1$-$C_6$-Alkylamino, $C_3$-$C_8$-Cycloalkylamino, Pyridyl, $C_1$-$C_4$-Alkylpyridyl, Pyrrolidinyl, Piperidinyl, N-$C_1$-$C_4$-Alkylpiperidinyl, $C_1$-$C_4$-Alkylcyclopropyl, Di-$C_1$-$C_4$-alkyl-dioxolanyl substituierten $C_1$-$C_{12}$-Alkyl-, $C_3$-$C_{12}$-Alkenyl- oder $C_3$-$C_{12}$-Alkinylrest,

$R^7$ ein Wasserstoffatom, einen gegebenenfalls durch Sauerstoff, Schwefel, die Imino- oder $C_1$-$C_4$-Alkyliminogruppe unterbrochenen und gegebenenfalls ein- oder mehrfach durch $C_1$-$C_4$-Alkyl, $C_1$-$C_4$-Alkoxy oder Halogen substituierten $C_3$-$C_8$-Cycloalkylrest, einen $C_5$-$C_8$-Cycloalkenylrest oder einen gegebenenfalls ein- oder mehrfach durch Cyano, Hydroxy, Mercapto, Amino, Hydroxy-$C_1$-$C_6$-alkyl, $C_1$-$C_6$-Alkoxy, $C_1$-$C_4$-Alkylthio, Carboxyl, Carbamoyl, Acetyl, $C_1$-$C_4$-Alkoxycarbonyl, $C_1$-$C_4$-Alkylthiocarbonyl, Oxo, Thioxo, Di-$C_1$-$C_3$-alkoxy, Hydroxyimino, $C_3$-$C_8$-Cycloalkyl, Dioxolanyl, $C_5$-$C_8$-Cycloalkenyl, Furyl, Tetrahydrofuryl, $C_1$-$C_6$-Alkylamino, $C_3$-$C_8$-Cycloalkylamino, Di-$C_1$-$C_6$-alkylamino, Pyranyl, Tetrahydropyranyl, Pyridyl, $C_1$-$C_4$-Alkylpyridyl, Pyrrolidinyl, Piperidinyl, N-$C_1$-$C_4$-Alkylpiperidinyl, $C_1$-$C_4$-Alkylcyclopropyl, Di-$C_1$-$C_4$-alkyldioxolanyl substituierten $C_1$-$C_{12}$-Alkyl-, $C_3$-$C_{12}$-Alkenyl- oder $C_3$-$C_{12}$-Alkinylrest,

B ein Wasserstoffatom und für den Fall, daß A = $COOR^3$ darstellt, wobei aber $R^3$ kein Wasserstoffatom bedeutet, auch die Gruppen $COR^8$ oder $SO^2R^9$,

$R^8$ einen $C_1$-$C_8$-Alkylrest, einen $C_1$-$C_6$-Halogenalkylrest oder einen gegebenenfalls ein- oder mehrfach durch Halogen, Nitro, Methyl oder Methoxy substituierten Phenylrest und

$R^9$ einen $C_1$-$C_6$-Alkylrest, einen Trifluormethylrest, einen Trichlormethylrest oder einen gegebenenfalls durch Halogen, Nitro oder $C_1$-$C_6$-Alkyl substituierten Phenylrest

bedeuten, überraschenderweise eine größere Wirksamkeit beziehungsweise eine verbesserte Selektivität aufweisen.

Die erfindungsgemäßen Verbindungen der allgemeinen Formel I lassen sich zum Beispiel herstellen, indem man

A) falls A = COOH und B = Wasserstoff bedeuten, eine Verbindung der allgemeinen Formel II

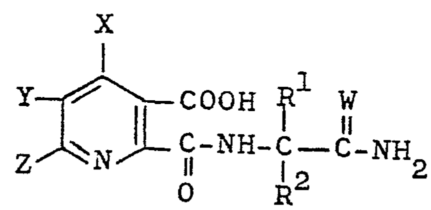

(II) ,

in der $R^1$, $R^2$, W, X, Y und Z die unter der allgemeinen Formel I angegebenen Bedeutungen haben, unter alkalischen Bedingungen cyclisiert,

B) falls A = $COOR^3$, $R^3$ aber nicht für ein Wasserstoffatom steht, und B = Wasserstoff bedeuten, eine Verbindung der allgemeinen Formel III

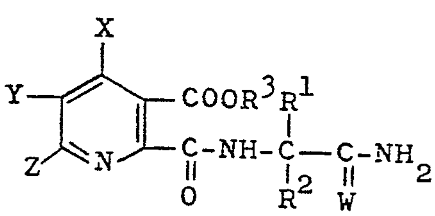

(III) ,

in der $R^1$, $R^2$, $R^3$, W, X, Y und Z die unter der allgemeinen Formel I angegebenen Bedeutungen haben, $R^3$ aber nicht für ein Wasserstoffatom steht, mit einem Gemisch aus Phosphoroxychlorid und Phosphorpentachlorid umsetzt,

C) falls A = $COOR^3$, $R^3$ aber nicht für ein Wasserstoffatom steht, $CONR^4R^5$ oder $CH_2OH$ und B = Wasserstoff bedeuten, ein Imidazopyrrolopyridin der allgemeinen Formel IV

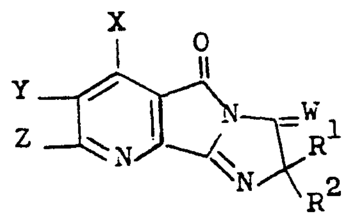

(IV) ,

in der $R^1$, $R^2$, W, X, Y und Z die unter der allgemeinen Formel I angegebenen Bedeutungen haben,

C1) mit einem Alkohol der allgemeinen Formel

$$R^3 - OH,$$

in der $R^3$ die unter der allgemeinen Formel I angegebenen Bedeutungen hat, aber nicht für ein Wasserstoffatom steht, und dem entsprechenden Alkalimetallalkoxid gegebenenfalls in Gegenwart eines Lösungsmittels umsetzt, oder

C2) mit einem Amin der allgemeinen Formel

$$HNR^4R^5,$$

in der $R^4$ und $R^5$ die unter der allgemeinen Formel I angegebenen Bedeutungen haben, gegebenenfalls unter Verwendung eines Lösungsmittels umsetzt, oder

C3) mit einem Reduktionsmittel, wie Natriumborhydrid, zur Reaktion bringt,

D) falls A = COOH bedeutet, eine Verbindung der allgemeinen Formel V

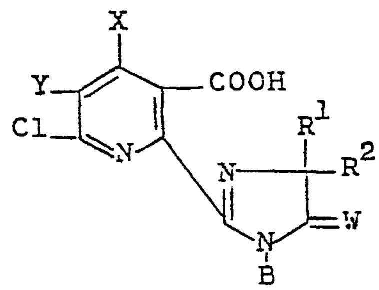

(V) ,

in der B, $R^1$, $R^2$, W, X und Y die unter der allgemeinen Formel I angegebene Bedeutung haben, mit einer Verbindung der allgemeinen Formel

ZH,

wobei Z die unter der allgemeinen Formel I angegebene Bedeutung hat, oder eine Verbindung der allgemeinen Formel XI

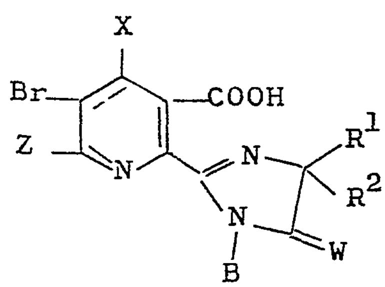

(XI),

in der B, $R^1$, $R^2$, W, X und Z die unter der allgemeinen Formel I angegebene Bedeutung haben, mit einer Verbindung der Formel

YH,

wobei Y die unter der allgemeinen Formel I angegebene Bedeutung hat, umsetzt,

E) falls $R^6$ oder $R^7$ im Substituenten Y oder Z nicht Wasserstoff bedeutet, eine Verbindung der allgemeinen Formel I, in der A, B, $R^1$, $R^2$, W, X, Y und Z die unter der allgemeinen Formel I angegebene Bedeutung haben, jedoch Y oder Z eine SH- oder OH-Gruppe darstellen, alkyliert,

F) gewünschtenfalls eine nach den Verfahrensvarianten A) und D) erhaltende Verbindung der allgemeinen Formel I, in der $R^3$ im Substituenten A für Wasserstoff steht, durch Umsetzung mit einem Äquivalent einer Base als Salzbildner in eine Verbindung der allgemeinen Formel I überführt, in der $R^3$ im Substituenten A für ein Kation steht, oder

G) falls B = $COR^8$ oder $SO_2R^9$ bedeutet, eine nach den Verfahrensvarianten B) oder C) erhaltene Verbindung der allgemeinen Formel I, in der A für $COOR^3$ steht, mit einem Acylhalogenid, Acylanhydrid oder Sulfonylhalogenid umsetzt.

Die Verfahrensvariante A) wird zweckmäßigerweise so durchgeführt, daß man das Ausgangsmaterial der allgemeinen Formel II über längere Zeit, wie 0,5 bis 20 Stunden, bei einer Temperatur im Bereich von 20 bis 100°C mit einer wäßrigen oder auch wäßrig-alkoholischen Alkalimetallhydroxidlösung, wie Natrium- oder Kaliumhydroxidlösung, behandelt, wobei die Menge des Hydroxids, bezogen auf das Ausgangsmaterial der allgemeinen Formel II, 2 bis 10 Moläquivalente beträgt. Anschließend wird das Reaktionsgemisch abgekühlt und mit einer starken Mineralsäure, wie Schwefel- oder Salzsäure, angesäuert.

Die als Ausgangsmaterial verwendeten Amide der allgemeinen Formel II lassen sich entsprechend dem folgenden Formelschema aus Verbindungen der allgemeinen Formel VI, in der X, Y und Z die unter der allgemeinen Formel I definierte Bedeutung haben, durch Überführen in ein Carbonsäureanhydrid der allgemeinen Formel VII nach üblichen Methoden und anschließender Umsetzung mit einem Aminosäureamid der allgemeinen Formel VIII, in dem $R^1$ und $R^2$ die unter der allgemeinen FormelI definierte Bedeutung haben, erhalten:

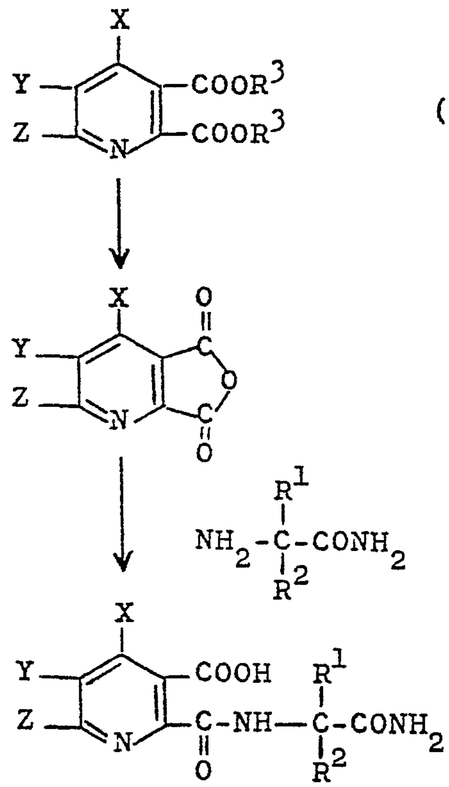

(VI)

(VII)

(VIII)

(II)

Die Herstellung von Verbindungen der allgemeinen Formel II erfolgt in Gegenwart eines organischen Lösungsmittels, wie zum Beispiel Diethylether, Tetrahydrofuran, Acetonitril oder Essigester, oder in einem halogenierten Lösungsmittel, wie zum Beispiel Methylenchlorid oder Chloroform. Die Reaktion findet in einem Temperaturbereich von 20° bis 100°C statt. Bei der Reaktion in geringen Mengen anfallende isomere Pyridincarbonsäurederivate lassen sich leicht durch Umkristallisation oder Chromatographie entfernen.

Die Ausgangsverbindungen der allgemeinen Formel VI sind zum Teil bekannt oder lassen sich nach an sich bekannten Methoden erhalten, zum Beispiel indem man 6-Chlorpyridin-2,3-dicarbonsäurederivate der allgemeinen Formel IX (X, Y = H, $R^3$ = $CH_3$, J. Chem. Soc. (B), 289-296 (1971)) oder 5-Halogenpyridin-2,3-dicarbonsäurederivate der allgemeinen Formel X (X, Z = H, Hal =

Cl, $R^3$ = $CH_3$; J. Med. Chem., 1067-71 (1974)) entsprechend folgendem Formelschema mit Verbindungen der allgemeinen Formel YH oder ZH umsetzt, und X, Y, Z und $R^3$ die unter der allgemeinen Formel I definierte Bedeutung haben:

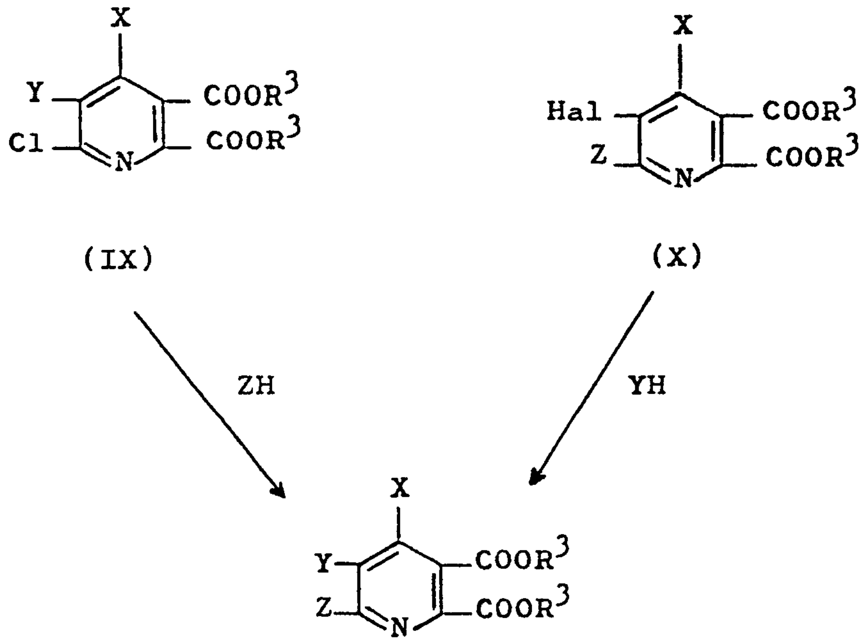


(IX) (X)

(VI)

Die Umsetzung erfolgt zwischen 0° und 150°C, im allgemeinen jedoch zwischen Raumtemperatur und der Rückflußtemperatur des Reaktionsgemisches. Die Reaktionsdauer beträgt 0,5 bis 48 Stunden. YH oder ZH werden zwischen 1 und 5 Mol, bezogen auf das Ausgangsmaterial, gegebenenfalls in Gegenwart einer geeigneten Base wie zum Beispiel Natriumhydrid, Natriumhydroxid, Kaliumhydroxid oder Kalium-tert.-butylat, umgesetzt. Die Umsetzung erfolgt ohne oder unter Verwendung geeigneter Lösungsmittel, wie zum Beispiel Dimethylsulfoxid, Halogenkohlenwasserstoffen, wie Methylenchlorid oder Chloroform, aromatischen Kohlenwasserstoffen, wie zum Beispiel Benzol, Toluol, Xylol, Chlorbenzol und Dichlorbenzol, Säureamiden, wie Dimethylformamid, oder anderen gegenüber den Reaktionspartnern inerten Lösungsmitteln, wie zum Beispiel Diethylether oder Tetrahydrofuran.

Die Verfahrensvariante B) wird zweckmäßigerweise so durchgeführt, daß man das Ausgangsmaterial der allgemeinen Formel III direkt mit einem Gemisch aus Phosphorpentachlorid und Phosphoroxychlorid bei Raumtemperatur zur Reaktion bringt.

Die Reaktion kann auch in Gegenwart eines unter den Reakionsbedingungen inerten Lösungsmittels, wie zum Beispiel Toluol, Xylol oder Chloroform, in einem Temperaturbereich von 0 bis 100°C durchgeführt werden. Die freien Basen werden aus den dabei anfallenden Hydrochloriden nach an sich üblichen Methoden, zum Beispiel durch Umsetzung mit Natriumcarbonat oder Natriumhydrogencarbonat, isoliert.

Das Ausgangsmaterial der allgemeinen Formel III wird aus Verbindungen der allgemeinen Formel II durch Veresterung nach an sich üblichen Methoden mit den entsprechenden Alkoholen der allgemeinen Formel $R^3$-OH erhalten.

Die Verfahrensvariante C1) wird zweckmäßigerweise so durchgeführt, daß man das Ausgangsmaterial der allgemeinen Formel IV mit einer Mischung aus Alkalimetallhydrid, wie Natriumhydrid, und dem entsprechenden Alkohol der Formel $R^3$-OH im Überschuß 0,5 bis 5 Stunden bei einer Temperatur im Bereich von Raumtemperatur bis zur Siedetemperatur des Reaktionsgemisches umsetzt. Der eingesetzte Alkohol dient bei dieser Umsetzung sowohl als Reaktand als auch als Lösungsmittel. Es können jedoch auch zusätzliche Lösungsmittel, wie zum Beispiel Tetrahydrofuran, Dioxan oder andere nicht protische Lösungsmittel, eingesetzt werden.

Die Verfahrensvariante C2) wird zweckmäßigerweise so durchgeführt, daß man das Ausgangsmaterial der allgemeinen Formel IV mit einem primären oder sekundären Amin der allgemeinen Formel

$$HNR^4R^5,$$

wobei $R^4$ und $R^5$ die unter der allgemeinen Formel I angegebenen Bedeutungen haben, in einem Temperaturbereich von 20 bis 100°C, gegebenenfalls unter Verwendung eines Lösungsmittels, wie zum Beispiel Dimethylformamid, Tetrahydrofuran oder Dioxan, zur Reaktion bringt.

Die Verfahrensvariante C3) wird zweckmäßigerweise so durchgeführt, daß man das Ausgangsmaterial der allgemeinen Formel IV mit einem Reduktionsmittel, wie Natriumborhydrid, in einem Temperaturbereich von -10 bis 30°C in einem geeigneten Lösungsmittel oder Lösungsmittelgemisch, wie zum Beispiel Ethanol/Tetrahydrofuran, zur Reaktion bringt.

Das Ausgangsmaterial der Formel IV läßt sich aus Verbindungen der allgemeinen Formel I, in der A = COOH und B = Wasserstoff bedeuten, durch Umsetzung mit Dicyclohexylcarbodiimid darstellen.

Die Umsetzung erfolgt in einem inerten Lösungsmittel, unter Verwendung einer äquimolaren Menge des Carbodiimids in einem Temperaturbereich von 20 bis 60°C.

Die Verfahrensvariante D) wird zweckmäßigerweise so durchgeführt, daß man das Ausgangsmaterial der allgemeinen Formel V in einem Lösungsmittel mit einer Verbindung Z-H, wobei Z die in der allgemeinen Formel I angegebene Bedeutung hat, bei Temperaturen zwischen 0 und 170°C, vorzugsweise zwischen Raumtemperatur und der Siedetemperatur des Reaktionsgemisches, umsetzt. Die Reaktion ist in Abhängigkeit von den Reaktanden nach 0,5 bis 48 Stunden beendet. Die Verbindungen der allgemeinen Formel Z-H, die an sich einen Alkohol oder ein Mercaptan darstellen, werden in einer Menge von 1 bis 5 Moläquivalenten, bezogen auf das Ausgangsmaterial der allgemeinen Formel V, gegebenenfalls in Gegenwart einer Base, wie zum Beispiel Natriumhydrid, Natriumhydroxid, Kaliumhydroxid oder Kalium-tert.-butylat, umgesetzt.

Die Umsetzung erfolgt ohne oder unter Verwendung eines geeigneten inerten Lösungsmittels. Als geeignete Lösungsmittel seien beispielsweise genannt Dimethylsulfoxid, Halogenkohlenwasserstoffe, wie zum Beispiel Methylenchlorid und Chloroform, aromatische Kohlenwasserstoffe, wie zum Beispiel Benzol, Toluol, Xylol, Chlorbenzol und Dichlorbenzol, sowie andere gegenüber den Reaktionspartnern inerte Lösungsmittel, wie zum Beispiel Diethylether, Tetrahydrofuran oder Dimethylformamid.

Die Ausgangsmaterialien der allgemeinen Formel V sind teilweise bekannt oder lassen sich nach bekannten Literaturmethoden darstellen.

Die Verfahrensvariante E) wird zweckmäßigerweise so durchgeführt, daß als Ausgangsmaterial eine Verbindung der allgemeinen Formel I, in welcher $R^6$ oder $R^7$ Wasserstoff ist, in an sich bekannter Weise alkyliert wird.

Hierzu wird das entsprechende 5-Hydroxy- oder 5-Mercapto- bzw. 6-Hydroxy- oder 6-Mercaptoderivat in einem geeigneten Lösungsmittel mit einem Alkylierungsmittel, vorzugsweise einem Alkylhalogenid, in Gegenwart einer Base bei Raumtemperatur umgesetzt. Geeignete Lösungsmittel sind beispielsweise Dimethylsulfoxid, Ether, wie zum Beispiel Tetrahydrofuran und Dioxan, Säureamide, wie Dimethylformamid und Dimethylacetamid, sowie Acetonitril.

Die Reaktion wird vorzugsweise bei Raumtemperatur ausgeführt, jedoch ist es auch möglich, bei erhöhter Temperatur zu arbeiten, wobei sich die Reaktionszeit naturgemäß verkürzt. Als Halogenide seien vorzugsweise die Chloride, Bromide und Jodide genannt. Geeignete Basen sind Alkalimetallhydride und -hydroxide, wie zum Beispiel Natriumhydrid und Kaliumhydroxid.

Zur Herstellung von Verbindungen der allgemeinen Formel I gemäß Verfahrensvariante F), deren Rest $R^3$ im Substituenten A ein Kation bedeutet, wird als Ausgangsmaterial eine Verbindung der allgemeinen Formel I eingesetzt, bei der $R^3$ für Wasserstoff steht. Diese wird in einem geeigneten Lösungsmittel mit einer Base als Salzbildner, wie zum Beispiel Alkalimetall- oder Erdalkalimetallhydroxid, Ammoniumhydroxid oder einem Alkylamin, wie zum Beispiel Isopropylamin, zweckmäßigerweise bei Raumtemperatur umgesetzt.

Gemäß Verfahrensvariante G) werden die entsprechenden nach den Verfahrensvarianten B) und C) synthetisierten Verbindungen der allgemeinen Formel I mit einer überschüsigen Menge eines Acylhalogenids der allgemeinen Formel $R^8$-CO-Cl, Acylanhydrids der allgemeinen Formel $R^8$-CO-O-CO-$R^8$ oder Sulfonylhalogenids der allgemeinen Formel Cl-$SO_2$-$R^9$, wobei $R^8$ und $R^9$ die unter der allgemeinen Formel I angegebene Bedeutung haben, gegebenenfalls in Gegenwart eines Lösungsmittels wie Pyridin oder Toluol, bei einer Temperatur zwischen 50 und 130°C umgesetzt.

Die nach den oben genannten Verfahrensvarianten hergestellten erfindungsgemäßen Verbindungen können nach üblichen Verfahren aus dem Reaktionsgemisch isoliert werden, beispielsweise indem man das Reaktionsgemisch in Eiswasser gibt und das Produkt durch Extraktion mit einem geeigneten Lösungsmittel oder durch Filtration abtrennt.

Die erfindungsgemäßen Verbindungen stellen in der Regel kristalline oder zähflüssige Substanzen dar, die zum Teil gut löslich in halogenierten Kohlenwasserstoffen, wie Chloroform, Sulfoxiden, wie Dimethylsulfoxid, oder Estern, wie Essigester, sind.

Die vorliegenden erfindungsgemäßen Verbindungen weisen eine ausgezeichnete herbizide Wirksamkeit gegen wirtschaftlich wichtige mono- und dikotyle Unkräuter auf. Auch schwer bekämpfbare perennierende Unkräuter werden durch die Wirkstoffe gut erfaßt.

Dabei ist es gleichgültig, ob die Substanzen in Vorsaat-, Vorauflauf- oder Nachauflaufspritzung ausgebracht werden. Werden die erfindungsgemäßen Verbindungen vor dem Keimen auf die Erdoberfläche appliziert, so wird das Auflaufen der Keimlinge nicht vollständig verhindert. Die Unkräuter wachsen bis zum Keimblattstadium heran, stellen jedoch dann ihr Wachstum ein und sterben schließlich ab.

Die erfindungsgemäßen Wirkstoffe können zum Beispiel gegen die folgenden Pflanzengattungen eingesetzt werden:

Dikotyle Unkräuter der Gattungen Abutilon, Chrysanthemum, Brassica, Helianthus, Mentha, Sinapis, Lepidium, Galium, Stellaria, Anthemis, Chenopodium, Atriplex, Senecio, Portulaca, Ipomoea, Matricaria, Galinsoga, Urtica, Amaranthus, Convolvulus, Polygonum, Sesbania, Ambrosia, Cirsium, Carduus, Sonchus, Solanum, Rorippa, Lamium, Veronica, Datura, Viola, Centaurea und Galeopsis.

Monokotyle Unkräuter der Gattungen Avena, Alopecurus, Echinochloa, Setaria, Panicum, Digitaria, Poa, Eleusine, Brachiaria, Lolium, Bromus, Cyperus, Agropyron, Sagittaria, Cynodon, Monochoria, Fimbristylis, Eleocharis, Ischaemum und Apera.

Die Verbindungen können in landwirtschaftlikchen Hauptkulturen wie Baumwolle, Soja, Reis, Mais, Weizen, Gerste, Hafer, Sorghum und Zuckerrohr angewendet werden.

Die Verwendung der erfindungsgemäßen Wirkstoffe ist keineswegs auf die genannten Unkrautgattungen und Kulturpflanzen beschränkt, sondern erstreckt sich in gleicher Weise auch auch auf andere Pflanzen.

Die Verbindungen eignen sich in Abhängigkeit von der Konzentration auch zur Totalunkrautbekämpfung, zum Beispiel auf Industrie- und Gleisanlagen und auf Wegen und Plätzen. Ebenso können die Verbindungen zur Unkrautbekämpfung in Dauerkulturen, wie zum Beispiel Forst-, Ziergehölz-, Obst-, Wein-, Citrus-, Nuß-, Bananen-, Kaffee-, Tee-, Gummi-, Ölpalm-, Kakao-, Beerenfrucht- und Hopfenanlagen, eingesetzt werden.

Darüber hinaus weisen die Verbindungen in entsprechend niederen Aufwandmengen wachstumsregulatorische Eigenschaften bei Kulturpflanzen auf, indem sie regulierend in den pflanzeneigenen Stoffwechsel eingreifen ohne die Pflanzen abzutöten, so daß sie zum Beispiel verwendet werden können zur generellen Steuerung und Hemmung von unerwünschten vegetativen Wachstum.

Die angewandte Wirkstoffmenge kann in einem größeren Bereich schwanken. Sie hängt im wesentlichen von der Art des gewünschten Effekts ab. Im allgemeinen liegen die Aufwandmengen zwischen 0,01 und 5 kg Wirkstoff pro Hektar Bodenfläche, vorzugsweise zum Beispiel bei der Unkrautbekämpfung zwischen 0,1 und 0,5 kg Wirkstoff pro Hektar.

Die erfindungsgemäßen Verbindungen können entweder allein, in Mischung miteinander oder mit anderen Wirkstoffen angewendet werden. Gegebenenfalls können andere Pflanzenschutz- oder Schädlingsbekämpfungsmittel je nach dem gewünschten Zweck zugesetzt werden.

Sofern eine Verbreiterung des Wirkungsspektrums beabsichtigt ist, können auch andere Herbizide zugesetzt werden. Beispielsweise eignen sich als herbizid wirksame Mischungspartner diejenigen Wirkstoffe, die in Weed Abstracts, Vol. 34, No.3, 1985, unter dem Titel "List of common names and abbreviations employed for currently used herbicides and plant growth regulators in Weed Abstracts" aufgeführt sind.

Eine Förderung der Wirkintensität und der Wirkungsgeschwindigkeit kann zum Beispiel durch wirkungssteigernde Zusätze, wie organische Lösungsmittel, Netzmittel und Öle, erzielt werden. Solche Zusätze lassen daher gegebenenfalls eine Verringerung der Wirkstoffdosierung zu.

Als Mischungspartner können außerdem Phospholipide verwendet werden, zum Beispiel solche aus der Gruppe Phosphatidylcholin, den hydrierten Phosphatidylcholinen, Phosphatidylethanolamin, den N-Acyl-phosphatidylethanolaminen, Phosphatidylinosit, Phosphatidylserin, Lysolecithin und Phosphatidylglycerol.

Zweckmäßig werden die gekennzeichneten Wirkstoffe oder deren Mischungen in Form von Zubereitungen, wie Pulvern, Streumitteln, Granulaten, Lösungen, Emulsionen oder Suspensionen, unter Zusatz von flüssigen und/oder festen Trägerstoffen beziehungsweise Verdünnungsmitteln und gegebenenfalls Haft-, Netz-, Emulgier- und/oder Dispergierhilfsmitteln angewandt.

Geeignete flüssige Trägerstoffe sind zum Beispiel aliphatische und aromatische Kohlenwasserstoffe wie Benzol, Toluol, Xylol, Cyclohexanon, Isophoron, Dimethylsulfoxid, Dimethylformamid, weiterhin Mineralölfraktionen und Pflanzenöle.

Als feste Trägerstoffe eignen sich Mineralien, zum Beispiel Bentonit, Silicagel, Talkum, Kaolin, Attapulgit, Kalkstein, und pflanzliche Produkte, zum Beispiel Mehle.

An oberflächenaktiven Stoffen sind zu nennen zum Beispiel Calciumligninsulfonat, Polyethylenalkylphenylether, Naphthalinsulfonsäuren und deren Salze, Phenolsulfonsäuren und deren Salze, Formaldehydkondensate, Fettalkoholsulfate sowie substituierte Benzolsulfonsäuren und deren Salze.

Der Anteil des beziehungsweise der Wirkstoffe(s) in den verschiedenen Zubereitungen kann in weiten Grenzen variieren. Beispielsweise enthalten die Mittel etwa 10 bis 90 Gewichtsprozent Wirkstoff, etwa 90 bis 10 Gewichtsprozent flüssige oder feste Trägerstoffe sowie gegebenenfalls bis zu 20 Gewichtsprozent oberflächenaktive Stoffe.

Die Ausbringung der Mittel kann in üblicher Weise erfolgen, zum Beispiel mit Wasser als Träger in Spritzbrühmengen von etwa 100 bis 1000 Liter/ha. Eine Anwendung der Mittel im sogenannten Low-Volume und Ultra-Low-Volume-Verfahren ist ebenso möglich wie ihre Applikation in Form von sogenannten Mikrogranulaten.

Die Herstellung dieser Zubereitungen kann in an sich bekannter Art und Weise, zum Beispiel durch Mahl- oder Mischverfahren, durchgeführt werden. Gewünschtenfalls können Zubereitungen der Einzelkomponenten auch erst kurz vor ihrer Verwendung gemischt werden, wie es zum Beispiel im sogenannten Tankmixverfahren in der Praxis durchgeführt wird.

Zur Herstellung der verschiedenen Zubereitungen werden zum Beispiel die folgenden Bestandteile eingesetzt:

**A) Spritzpulver**

1.) 80 Gewichtsprozent Wirkstoff
15 Gewichtsprozent Kaolin
5 Gewichtsprozent oberflächenaktive Stoffe auf Basis des Natriumsalzes des N-Methyl-N-oleyl-taurins und des Calciumsalzes der Ligninsulfonsäure

2.) 20 Gewichtsprozent Wirkstoff
35 Gewichtsprozent Bentonit
8 Gewichtsprozent Calciumsalz der Ligninsulfonsäure
2 Gewichtsprozent Natriumsalz des N-Methyl-N-oleyl-taurins
35 Gewichtsprozent Kieselsäure

B) Paste

| | |
|---|---|
| 45 Gewichtsprozent | Wirkstoff |
| 5 Gewichtsprozent | Natriumaluminiumsilikat |
| 15 Gewichtsprozent | Cetylpolyglycolether mit 8 Mol Ethylenoxid |
| 2 Gewichtsprozent | Spindelöl |
| 10 Gewichtsprozent | Polyethylenglycol |
| 23 Gewichtsprozent | Wasser |

C) Emulsionskonzentrat

| | |
|---|---|
| 20 Gewichtsprozent | Wirkstoff |
| 50 Gewichtsprozent | Cyclohexanon |
| 25 Gewichtsprozent | aromatische Kohlenwasserstoffe |
| 5 Gewichtsprozent | Kombinationsemulgator aus Calciumdodecylbenzolsulfonat und Nonylphenolpolyglycolether |

Die folgenden Beispiele erläutern die Herstellung der erfindungsgemäßen Verbindungen:

**Beispiel 1** (Verfahren A)

6-Benzylthio-2-(4-isopropyl-4-methyl-5-oxo-2-imidazolin-2-yl)-nicotinsäure

7,5 g (0,028 mol) 6-Benzylthiopyridin-2,3-dicarbonsäureanhydrid werden mit 30 ml Acetonitril aufgeschlämmt und unter Rühren mit einer Lösung aus 3,6 g (0,028 mol) 2-Methyl-valinamid in 20 ml Acetonitril versetzt. Nach 15 Minuten wird eingeengt und die gebildete 6-Benzylthio-2-(N-1-carbamoyl-1,2-dimethylpropyl-carbamoyl)-nicotinsäure mit 50 ml 2,6 N Natriumhydroxid versetzt und 6 Stunden unter Rühren auf 100°C erwärmt. Bei Raumtemperatur wird mit konzentrierter Salzsäure angesäuert, mit Methylenchlorid extrahiert, über Magnesiumsulfat getrocknet und eingeengt.

Ausbeute: 3,6 g (33,5 % der Theorie)
Fp.: 190°C

Das Ausgangsmaterial wurde wie folgt hergestellt:

6-Benzylthiopyridin-2,3-dicarbonsäuredimethylester
3,7 g (0,03 mol) Benzylmercaptan werden zu einer Mischung aus 8,3 g (0,06 mol) Kaliumcarbonat in 20 ml Dimethylformamid gegeben und 10 Minuten gerührt. Anschließend werden 6,9 g (0,03 mol) 6-Chlorpyridin-2,3-dicarbonsäuredimethylester zugegeben und das Reaktionsgemisch 6 Stunden bei 100°C gerührt. Nach dem Abkühlen auf Raumtemperatur wird das Reaktionsgemisch in Eiswasser gegeben, das Produkt mit Methylenchlorid extrahiert, die organische Phase mit verdünnter Salzsäure und Wasser gewaschen, über Magnesiumsulfat getrocknet und einrotiert. Aus dem öligen Rückstand kristallisiert das Produkt aus.

Ausbeute: 7,3 g (76,8 % der Theorie)
Fp.: 80-84°C

6-Benzylthiopyridin-2,3-dicarbonsäure

15,0 g (0,375 mol) Natriumhydroxid werden in 40 ml Wasser gelöst und zu einer gerührten Mischung aus 7,3 g (0,023 mol) 6-Benzylthiopyridin-2,3-dicarbonsäuredimethylester in 40 ml Ether gegeben. Es wird 4 Stunden bei 40°C gerührt, die wäßrige Phase mit konzentrierter Salzsäure angesäuert und das Produkt mit Essigester extrahiert, das Lösungsmittel über Magnesiumsulfat getrocknet und eingeengt. Aus dem Rückstand kristallisiert das Produkt aus.

Ausbeute: 6,36 g (95,6 % der Theorie)
Fp.: 175°C

6-Benzylthio-pyridin-2,3-dicarbonsäureanhydrid

8,5 g (0,029 mol) 6-Benzylthiopyridin-2,3-dicarbonsäure werden in 150 ml Acetanhydrid 6 Stunden bei 110°C gerührt. Die Substanz löst sich dabei vollständig. Das Lösungsmittel wird im Vakuum abdestilliert und der Rückstand ohne weitere Reinigung in die nächste Stufe eingesetzt.

Ausbeute: 7,5 g (78,6 % der Theorie)

**Beispiel 2** (Verfahren F)

6-Benzylthio-2-(4-isopropyl-4-methyl-5-oxo-2-imidazolin-2-yl-nicotinsäure, Isopropylammoniumsalz

Zu einer Lösung aus 1,0 g (2,7 mmol) 6-Benzylthio-2-(4-isopropyl-4-methyl-5-oxo-2-imidazolin-2-yl)-nicotinsäure in 50 ml Ether und 50 ml Tetrahydrofuran werden 0,16 g (2,7 mmol) Isopropylamin gegeben, der Niederschlag abgesaugt und getrocknet.

Ausbeute: 0,93 g (80,75 % der Theorie)
Fp.: 174-175°C

**Beispiel 3** (Verfahren B)

5-Hydroxy-2-(4-isopropyl-4-methyl-5-oxo-2-imidazolin-2-yl)-6-methyl-nicotinsäuremethylester

Eine Lösung aus 47 g (0,129 mol) 5-Acetoxy-2-(1-carbamoyl-1,2-dimethylpropyl-carbamoyl)-6-methyl-nicotinsäuremethylester und 93,8 g Phosphorpentachlorid in 470 ml Phosphoroxychlorid wird 12 Stunden bei Raumtemperatur gerührt. Danach hydrolysiert man mit Eis und neutralisiert die Lösung mit Natriumcarbonat. Das Produkt wird mit Essigester extrahiert, die Extrakte über Magnesiumsulfat getrocknet und eingeengt. Das Rohprodukt wird aus Essigester/Isopropylester umkristallisiert.

Ausbeute: 22 g (55,9 % der Theorie)
Fp.: 206°C

Das Ausgangsmaterial wurde wie folgt hergestellt:

5-Acetoxy-6-methyl-pyridin-2,3-dicarbonsäureanhydrid
Eine Suspension aus 1,0 g (5,1 mmol) 5-Hydroxy-6-methylpyridin-2,3-dicarbonsäure und 5 ml Essigsäureanhydrid werden bei einer Badtemperatur von 140°C so lange erwärmt, bis eine klare Lösung entsteht. Nach dem Einengen erhält man das gewünschte Produkt.

Ausbeute: 1,1 g (97,5 % der Theorie)
Fp.: 123-125°C.

5-Acetoxy-2-(1-carbamoyl-1,2-dimethylpropyl-carbamoyl)-6-methyl-nicotinsäure
6,3 g (28,48 mmol) 5-Acetoxy-6-methyl-pyridin-2,3-dicarbonsäureanhydrid und 4,1 g (31,33 mmol) 2-Methyl-valinamid werden unter Stickstoff 2 Tage bei Raumtemperatur gerührt und anschließend das ausgefallene kristalline Produkt abfiltriert.

Ausbeute: 9,5 g (95 % der Theorie)
Fp.: ab 143°C

5-Acetoxy-2-(1-carbamoyl-1,2-dimethylpropyl-carbamoyl)-6-methyl-nicotinsäuremethylester

Durch eine unter Rückfluß siedende Lösung aus 53 g (0,151 mol) 5-Acetoxy-2-(1-carbamoyl-1,2-dimethylpropyl-carbamoyl)-6-methylnicotinsäure in 500 ml absolutem Methanol leitet man für 3 Stunden Salzsäuregas. Anschließend wird das Lösungsmittel abgedampft und das Produkt im Vakuum getrocknet.

Ausbeute: 47,1 g (85,5 % der Theorie)
Fp.: 145°C Zers.

**Beispiel 4** (Verfahren C)

6-Benzylthio-2-(4-isopropyl-4-methyl-5-oxo-2-imidazolin-2-yl)-nicotinsäuremethylester

4,0 g (0,011 mol) 2-Isopropyl-2-methyl-5H-imidazo[1',2':1,2]-pyrrolo[3,4-b]-2-benzylthiopyridin-3(2H)-5-dion wird zu einer Mischung aus 0,2 g Natriumhydrid in 40 ml Methanol gegeben und 2 Stunden unter Rückfluß erwärmt. Die Mischung wird nach dem Abkühlen in Eiswasser gegeben und das Produkt mit Chloroform extrahiert.

Ausbeute: 2,7 g (61,8 % der Theorie)
Fp.: 157-158°C.

Das Ausgangsmaterial wurde wie folgt hergestellt:

2-Isopropyl-2-methyl-5H-imidazo[1',2':1,2]-pyrrolo[3,4-b]-2-benzylthiopyridin-3(2H)-5-dion

1,0 g (0,005 mol) Dicyclohexylcarbodiimid und 2,0 g (0,005 mol) 6-Benzylthio-2-(4-isopropyl-4-methyl-5-oxo-2-imidazolin-2-yl)-nicotinsäure werden in 30 ml Methylenchlorid gelöst, 4 Stunden bei Raumtemperatur gerührt und über Nacht stehengelassen. Das Reaktionsgemisch wird filtriert und das Filtrat anschließend eingeengt. Der ölige Rückstand kristallisiert nach einiger Zeit.

Ausbeute: 1,7 g (93,4 % der Theorie)
Fp.: 155-157 °C

**Beispiel 5 (Verfahren D)**

2-(4-Isopropyl-4-methyl-5-oxo-2-imidazolin-2-yl)-6-tetrahydrofurfuryloxy-nicotinsäure

1,0 ml (0,01 mol) Tetrahydrofurfurylalkohol werden zu einer Mischung aus 1,0 g (0,033 mol) Natriumhydrid (80 %ig) in 10 ml Dimethylformamid gegeben und 1 Stunde nachgerührt. Anschließend werden 2,95 g (0,01 mol) 6-Chlor-2-(4-isopropyl-4-methyl-5-oxo-2-imidazolin-2-yl)-nicotinsäure, gelöst in 10 ml Dimethylformamid, zugetropft. Man rührt für 1 Stunde bei 50 bis 60 °C, kühlt auf Raumtemperatur ab, gibt 10 ml Wasser hinzu und säuert mit konzentrierter Salzsäure an. Das Produkt wird mit Methylenchlorid extrahiert, der Extrakt über Magnesiumsulfat getrocknet, filtriert und eingeengt. Aus dem erhaltenen Öl kristallisiert nach einiger Zeit das Produkt aus.

Ausbeute: 1,8 g (50 % der Theorie)
Fp.: 155-160 °C.

**Beispiel 6 (Verfahren E)**

2-(4-Isopropyl-4-methyl-5-oxo-2-imidazolin-2-yl)-6-methyl-5-methylthiomethoxy-nicotinsäuremethylester

Eine Lösung aus 2,5 g (8,19 mmol) 5-Hydroxy-2-(4-isopropyl-4-methyl-5-oxo-2-imidazolin-2-yl)-6-methyl-nicotinsäuremethylester in 25 ml Dimethylsulfoxid wird mit 0,26 g (8,19 mmol) Natriumhydrid (80 %ig) versetzt und nach 10 Minuten Rühren werden 0,87 g (9,01 mmol) Chlordimethylsulfid zugegeben. Es wird über Nacht gerührt, anschließend auf 500 ml Eiswasser gegeben, das Produkt mit Essigester extrahiert, die Extrakte über Magnesiumsulfat getrocknet und eingeengt. Der Rückstand wird über Kieselgel mit Essigester/Hexan 1 : 1 chromatographiert.

Ausbeute: 0,53 g (17,7 % der Theorie)
Fp.: 96-98°C.

Analog wurden die in der folgenden Tabelle formelmäßig aufgeführten Verbindungen der Formel I nach den bezeichneten Verfahrensvarianten hergestellt:

Tabelle

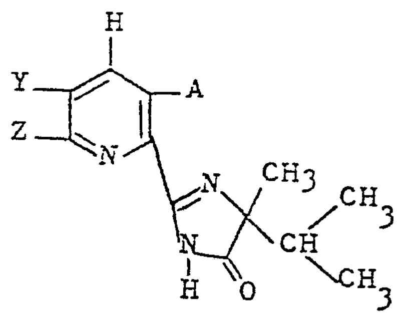

| Beispiel Nr. | Y | Z | A | Fp. [°C]. | Verfahrens-variante |
|---|---|---|---|---|---|
| 7 | H | 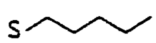 | COOH | 105-108 | D |
| 8 | H | 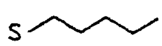 | COOH x 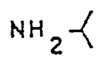 | Öl | F |
| 9 | H | 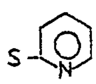 | COOH | 155 | D |
| 10 | H | 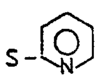 | COOH x 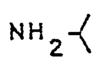 | 91-95 | F |
| 11 | H | O-$CH_2$-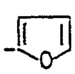 | COOH | 208 | D |
| 12 | H | O-$CH_2$-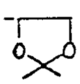 | COOH | 164 | D |
| 13 | H | S-$(CH_3)_2$Ph | COOH x $H_2O$ | 150 | D |
| 14 | H | S-$CH_2$-Ph | $COOC_2H_5$ | 126-128 | C |

0216360

| Beispiel Nr. | Y | Z | A | Fp. [°C] | Verfahrens-variante |
|---|---|---|---|---|---|
| 15 | H | S-$CH_2$-Ph | COO-i-$C_3H_7$ | 175-177 | C |
| 16 | H | S-$CH_2$-Ph | COO-$CH_2$-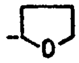 | 100 | C |
| 17 | H | S-$CH_2$-Ph | COO-$CH_2$-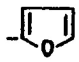 | 138 | C |
| 18 | H | S-$CH_2$-Ph | COO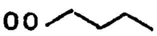 | 140 | C |
| 19 | H | S-$CH_2$-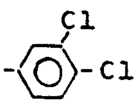-Cl | COOH | 171 | D |
| 20 | H | S-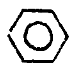-F | COOH | 160 | D |
| 21 | H | S-$CH_2$-CH=$CH_2$ | COOH | 155-160 | D |
| 22 | H | S-$CH_2$-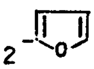 | COOH | 170-180 | D |
| 23 | H | S-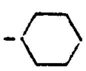 | COOH | 125-129 | D |
| 24 | H | S-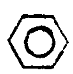 | COOH | 136 | D |
| 25 | H | S-$(CH_2)_2$Ph | COOH | 122 | D |
| 26 | H | S-$(CH_2)_2$-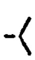 | COOH | 120 | D |
| 27 | H | S-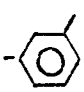 | COOH | 115 | D |
| 28 | H | S-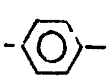 | COOH | 120 | D |
| 29 | H | S-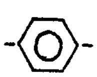-Cl | COOH | 158 | D |
| 30 | H | S-$(CH_2)_2$-OH | COOH | 180 | D |
| 31 | H | O-$CH_2$-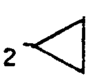 | COOH | 170 | D |

| Beispiel Nr. | Y | Z | A | Fp. [°C] | Verfahrens-variante |
|---|---|---|---|---|---|
| 32 | H | O-$CH_2$-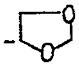 | COOH | 130 | D |
| 33 | H | O-$CH_2$-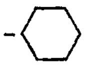 | COOH | 142 | D |
| 34 | H | O-$CH_2$-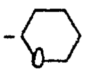 | COOH | 180 | D |
| 35 | H | SH | COOH | 175/Zers. | D |
| 36 | H | S-$CH_2$-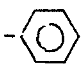 | $CONH_2$ | 198-204 | C |
| 37 | H | S-$CH_2$-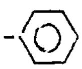 | $CONHNH_2$ | >250 | C |
| 38 | H | S-$CH_2$-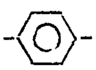-Cl | COOH | 130-140 | D |
| 39 | H | S-$CH_2$-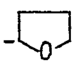 | COOH | 95 | D |
| 40 | H | S-$CH_2$-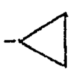 | COOH | 140 | D |
| 41 | $CH_3$ | O-$CH_2$-S-$CH_3$ | COOH | 146-150 | E |
| 42 | H | S-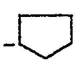 | COOH | 196 | D |
| 43 | H | O-$CH_2$-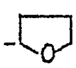 | COOH x 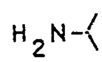 | 157/Zers. | F |
| 44 | H | O-$CH_2$-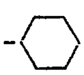 | COOH x 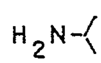 | 169/Zers. | F |
| 45 | H | S-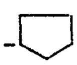 | COOH x 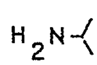 | 158/Zers. | F |
| 46 | H | S-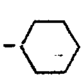 | COOH x 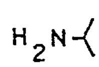 | 167/Zers. | F |
| 47 | H | O-$CH_2$-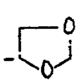 | COOH x 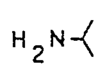 | 146/Zers. | F |
| 48 | H | O-$CH_2$-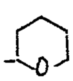 | COOH x 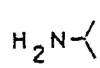 | 151/Zers. | F |

| Beispiel Nr. | Y | Z | A | Fp. [°C] | Verfahrens-variante |
|---|---|---|---|---|---|
| 49 | H | S-$CH_2$-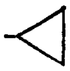 | COOH x $H_2N$-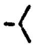 | 158/Zers. | F |
| 50 | H | S-$CH_2$-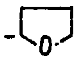 | COOH x $H_2N$-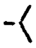 | 144/Zers. | F |
| 51 | H | S-$CH_2$-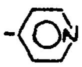 | COOH | 208/Zers. | D |
| 52 | S-$CH_2$-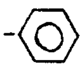 | H | COOH | 112 | D |
| 53 | S-$CH_2$-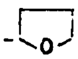 | H | COOH | 130 | D |
| 54 | S-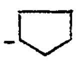 | H | COOH | 141 | D |
| 55 | O-$CH_2$-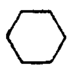 | H | COOH | 158 | D |
| 56 | O-$CH_2$-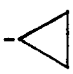 | H | COOH | 110 | D |
| 57 | S-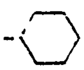 | H | COOH | 154 | D |
| 58 | H | O-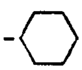 | COOH | 189-191 | D |
| 59 | H | O-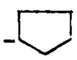 | COOH | 188-192 | D |
| 60 | H | O-$CH_2$-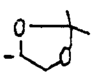 | COOH x $H_2N$-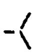 | 157/Zers. | F |
| 61 | H | O-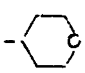 | COOH | 145 | D |
| 62 | H | O-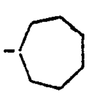 | COOH | 140-143 | D |
| 63 | H | O-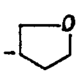 | COOH | 185 | D |
| 64 | H | O-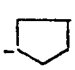 | COOH x $H_2N$-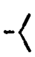 | 182/Zers. | F |
| 65 | H | O-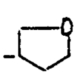 | COOH x $H_2N$-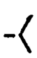 | 157/Zers. | F |
| 66 | O-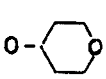 | H | COOH | 150-156 | D |

Beispiel 67 (Verfahren D)

5-(Cyclopropylmethoxy)-2-(4-isopropyl-4-methyl-5-oxo-2-imidazolin-2-yl)-nicotinsäure, Hydrat

1,0 g (0,033 mol) 80 %iges Natriumhydrid wird in 15 ml Dimethylformamid gelöst und mit 1,0 g (0,015 mol) Cyclopropylmethylalkohol versetzt. Unter Rühren werden 3,4 g (0,01 mol) 5-Brom-1-(4-isopropyl-4-methyl-5-oxo-2-imidazolin-2-yl)-nicotinsäure hinzugegeben und 2 Stunden bei 100°C Badtemperatur gerührt. Nach dem Abkühlen wird das Reaktionsgemisch auf Eis gegeben, mit konzentrierter Salzsäure angesäuert und mit Essigester extrahiert. Die organische Phase wird über Magnesiumsulfat getrocknet und eingeengt. Der ölige Rückstand kristallisiert aus Isopropylether.

Ausbeute: 1,7 g (48,6 % der Theorie)
Fp.: 105-110°C

Die folgenden Beispiele dienen zur Erläuterung der Anwendungsmöglichkeiten der erfindungsgemäßen Verbindungen, die in Form der oben genannten Zubereitungen erfolgte:

**Beispiel A**

Im Gewächshaus wurden die erfindungsgemäßen Verbindungen in einer Aufwandmenge von 3 kg Wirkstoff/ha emulgiert in 100 l Wasser/ha auf Brassica spp. und Solanum spp. als Testpflanzen im Vor- und Nachauflauf-Verfahren gespritzt. 3 Wochen nach der Behandlung wurde das Behandlungsergebnis bonitiert, wobei folgendes Schema verwendet wurde:

0 = keine Wirkung
1 = geringe Wirkung oder schwache Inhibition des Pflanzenwuchses
2 = mittlere Wirkung oder Inhibition des Pflanzenwuchses
3 = totale Wuchsinhibition
4 = totale Vernichtung der Pflanzen.

Es ergab sich, daß die Verbindungen der Beispiele 1, 2, 5-8, 11-13, 19-26, 33-59 und 66 in diesem Test eine 100 %ige Vernichtung (= 4) der Pflanzen bewirkten.

**Beispiel B**

Im Gewächshaus wurden die aufgeführten Pflanzen vor dem Auflaufen mit der zu untersuchenden Verbindung in einer Aufwandmenge von 1 kg Wirkstoff/ha behandelt. Die Verbindung wurde zu diesem Zweck als Suspension mit 500 l Wasser/ha gleichmäßig über den Boden versprüht. 3 Wochen nach der Behandlung zeigten die erfindungsgemäßen Verbindungen eine Selektivität in Mais bei ausgezeichneter Wirkung gegen das Unkraut. Das Vergleichsmittel war deutlich weniger wirksam.

In der nachfolgenden Tabelle bedeuten:

0 = keine Wirkung

1 = geringe Wirkung oder schwache Inhibition des Pflanzenwuchses

2 = mittlere Wirkung oder Inhibiton des Pflanzenwuchses

3 = totale Wuchsinhibition

4 = totale Vernichtung der Pflanzen

Ze = Zea mays
Br = Brassica sp.
So = Solanum sp.
Me = Medicago sp.
He = Helianthus sp.
St = Stellaria sp.
Ab = Abutilon sp.
Ma = Matricaria sp.
Vi = Viola sp.
Ch = Chrysanthemum sp.
Sr = Sorghum sp.
Av = Avena sp.
Al = Alopecurus sp.
Ec = Echinochloa sp.
Se = Setaria sp.
Cy = Cyperus sp.

| Erfindungsgemäße Verbindungen | Ze | Br | So | Me | He | St | Ab | Ma | Vi | Ch | Sr | Av | Al | Ec | Se | Cy |
|---|---|---|---|---|---|---|---|---|---|---|---|---|---|---|---|---|
| Beispiel 5 | 0 | 3 | 3 | 3 | 3 | 3 | 4 | 4 | 3 | 3 | 4 | 4 | 4 | 4 | 4 | 4 |
| Beispiel 12 | 0 | 3 | 3 | 3 | 3 | 3 | 3 | 4 | 3 | 3 | 3 | 3 | 3 | 3 | 4 | 3 |
| **Vergleichsmittel** (gemäß DE-OS 31 21 736) | | | | | | | | | | | | | | | | |
| 6-Butylthio-2-(4-isopropyl-4-methyl-5-oxo-2-imidazolin-2-yl)-nicotinsäure | 0 | 2 | 2 | 0 | 1 | 0 | 2 | 4 | 2 | 1 | 0 | 1 | 1 | 0 | 1 | 2 |

Beispiel C

Im Gewächshaus wurden die aufgeführten Pflanzen vor dem Auflaufen mit den aufgeführten Verbindungen in einer Aufwandmenge von 1 kg Wirkstof/ha behandelt. Die Verbindungen wurde zu diesem Zweck als Suspension mit 500 Liter Wasser/ha gleichmäßig über den Boden versprüht. 3 Wochen nach der Behandlung zeigten die erfindungsgemäßen Verbindungen eine Selektivität in Soja (Glycine max.) bei ausgezeichneter Wirkung gegen das Unkraut.

In der nachfolgenden Tabelle bedeuten:

0 = keine Wirkung
1 = geringe Wirkung oder schwache Inhibition des Pflanzenwuchses
2 = mittlere Wirkung oder Inhibition des Pflanzenwuchses
3 = totale Wuchsinhibition
4 = totale Vernichtung der Pflanzen

Gl = Glycine max.
Br = Brassica sp.
So = Solanum sp.
Me = Medicago sp.
He = Helianthus sp.
St = Stellaria sp.
Ma = Matricaria sp.
Ch = Chrysanthemum sp.
Se = Setaria sp.

| Erfindungsgemäße Verbindungen | Gl | Br | So | Me | He | St | Ma | Ch | Se |
|---|---|---|---|---|---|---|---|---|---|
| Beispiel 58 | 0 | 4 | 4 | 4 | 4 | 4 | 4 | 4 | 4 |
| Beispiel 59 | 0 | 4 | 4 | 4 | 4 | 4 | 4 | 4 | 4 |

0216360

**Patentansprüche für die Vertragsstaaten: BE, CH, DE, FR, GB, IT, LU, NL, SE**

1. 2-(2-Imidazolin-2-yl)-pyridin-3-carbonsäurederivate der allgemeinen Formel I

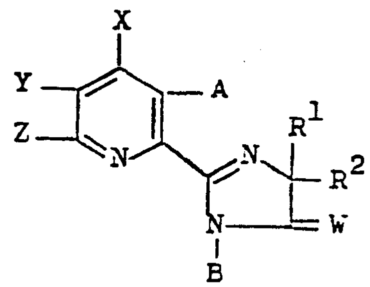

(I) ,

in der

$R^1$ einen $C_1$-$C_4$-Alkylrest,

$R^2$ einen $C_1$-$C_6$-Alkylrest oder einen $C_3$-$C_6$-Cycloalkylrest oder

$R^1$ und $R^2$ gemeinsam mit dem benachbarten Kohlenstoffatom eigegebenenfalls durch Methyl substituierten $C_3$-$C_6$-Cycloalkylrest,

W ein Sauerstoff- oder Schwefelatom,

A eine der Gruppen $CH_2OH$, $COOR^3$ oder $CONR^4R^5$,

$R^3$ ein Wasserstoffatom, einen gegebenenfalls durch ein oder mehrere Sauerstoff- oder Schwefelatome unterbrochenen und gegebenenfalls durch $C_1$-$C_3$-Alkoxy, Halogen, Hydroxy, $C_3$-$C_6$-Cycloalkyl, Benzyl, Furyl, Tetrahydrofuryl, Phenyl, Halgenphenyl, $C_1$-$C_4$-Alkylphenyl, $C_1$-$C_4$-Alkoxyphenyl, Nitrophenyl, Cyano, Carboxyl, $C_1$-$C_4$-Alkoxycarbonyl oder $C_1$-$C_4$-Alkylthio substituierten $C_1$-$C_{12}$-Alkylrest, einen gegebenenfalls durch $C_1$-$C_3$-Alkoxy, Phenyl oder Halogen substituierten $C_3$-$C_8$-Alkenylrest, einen gegebenenfalls durch $C_1$-$C_3$-Alkoxy, Phenyl oder Halogen substituierten-$C_3$-$C_8$-Alkinylrest, einen gegebenenfalls durch Methyl substituierten $C_3$-$C_6$-Cycloalkylrest oder ein Kation aus der Gruppe der Alkalimetalle, Erdalkalimetalle, Mangan, Kupfer, Eisen, Zink, Ammonium und organische Ammoniumverbindungen,

$R^4$ und $R^5$ unabhängig voneinander ein Wasserstoffatom, eine Hydroxygruppe oder einen $C_1$-$C_4$-Alkylrest und für den Fall daß $R^4$ ein Wasserstoffatom ist, $R^5$ auch einen Amino-, Dimethylamino, Acetylamino- oder Anilinorest,

X ein Wasserstoffatom, eine Nitrogruppe, einen $C_1$-$C_3$-Alkylrest, einen $C_1$-$C_3$-Halogenalkylrest oder einen $C_1$-$C_3$-Alkoxyrest und einer der beiden Reste

Y oder Z eine der Gruppen $SR^6$ oder $OR^7$ und der jeweils andere ein Wasserstoffatom, ein Halogenatom, einen $C_1$-$C_4$-Alkylrest, einen $C_1$-$C_4$-Halogenalkylrest, einen $C_1$-$C_4$-Alkoxyrest oder einen $C_1$-$C_4$-Alkylthiorest,

$R^6$ ein Wasserstoffatom, einen Phenylrest, einen Halogenphenylrest, einen $C_1$-$C_3$-Alkylphenylrest, einen Di-$C_1$-$C_3$-alkylphenylrest, einen Nitrophenylrest, einen $C_1$-$C_3$-Alkoxyphenylrest, einen Pyridylrest, einen $C_1$-$C_3$-Alkyl-pyridylrest, einen ein- oder mehrfach durch Sauerstoff unterbrochenen $C_2$-$C_8$-Alkylrest, einen gegebenenfalls durch Sauerstoff, Schwefel, die Imino- oder $C_1$-$C_4$-Alkyliminogruppe unterbrochenen und gegebenenfalls ein- oder mehrfach durch $C_1$-$C_4$-Alkyl, $C_1$-$C_4$-Alkoxy oder Halogen substituierten $C_3$-$C_8$-Cycloalkylrest, einen $C_5$-$C_8$-Cycloalkenylrest oder einen gegebenenfalls ein- oder mehrfach durch Cyano, Halogen, Hydroxy, Mercapto, Amino, $C_1$-$C_6$-Alkoxy, $C_1$-$C_4$-Alkylthio, Carboxyl, Carbamoyl, $C_1$-$C_4$-Alkoxycarbonyl, $C_1$-$C_4$-Alkylthiocarbonyl, Oxo, Thioxo, Di-$C_1$-$C_3$-alkoxy, Hydroxyimino, $C_3$-$C_8$-Cycloalkyl, $C_3$-$C_8$-Cycloalkenyl, Phenyl, Halogenphenyl, $C_1$-$C_3$-Alkylphenyl, Nitrophenyl, $C_1$-$C_3$-Alkoxyphenyl, Furyl, Tetrahydrofuryl, Dioxolanyl, $C_1$-$C_6$-Alkylamino, $C_3$-$C_8$-Cycloalkylamino, Pyridyl, $C_1$-$C_4$-Alkylpyridyl, Pyrrolidinyl, Piperidinyl, N-$C_1$-$C_4$-Alkylpiperidinyl, $C_1$-$C_4$-Alkylcyclopropyl, Di-$C_1$-$C_4$-alkyl-dioxolanyl substituierten $C_1$-$C_{12}$-Alkyl-, $C_3$-$C_{12}$-Alkenyl- oder $C_3$-$C_{12}$-Alkinylrest,

$R^7$ ein Wasserstoffatom, einen gegebenenfalls durch Sauerstoff, Schwefel, die Imino- oder $C_1$-$C_4$-Alkyliminogruppe unterbrochenen und gegebenenfalls ein- oder mehrfach

durch $C_1$-$C_4$-Alkyl, $C_1$-$C_4$-Alkoxy oder Halogen substituierten $C_3$-$C_8$-Cycloalkylrest, einen $C_5$-$C_8$-Cycloalkenylrest oder einen gegebenenfalls ein- oder mehrfach durch Cyano, Hydroxy, Mercapto, Amino, Hydroxy-$C_1$-$C_6$-alkyl, $C_1$-$C_6$-Alkoxy, $C_1$-$C_4$-Alkylthio, Carboxyl, Carbamoyl, Acetyl, $C_1$-$C_4$-Alkoxycarbonyl, $C_1$-$C_4$-Alkylthiocarbonyl, Oxo, Thioxo, Di-$C_1$-$C_3$-alkoxy, Hydroxyimino, $C_3$-$C_8$-Cycloalkyl, Dioxolanyl, $C_5$-$C_8$-Cycloalkenyl, Furyl, Tetrahydrofuryl, $C_1$-$C_6$-Alkylamino, $C_3$-$C_8$-Cycloalkylamino, Di-$C_1$-$C_6$-alkylamino, Pyranyl, Tetrahydropyranyl, Pyridyl, $C_1$-$C_4$-Alkylpyridyl, Pyrrolidinyl, Piperidinyl, N-$C_1$-$C_4$-Alkylpiperidinyl, $C_1$-$C_4$-Alkylcyclopropyl, Di-$C_1$-$C_4$-alkyldioxolanyl substituierten $C_1$-$C_{12}$-Alkyl-, $C_3$-$C_{12}$-Alkenyl- oder $C_3$-$C_{12}$-Alkinylrest,

B ein Wasserstoffatom und für den Fall, daß A = $COOR^3$ darstellt, wobei aber $R^3$ kein Wasserstoffatom bedeutet, auch die Gruppen $COR^8$ oder $SO^2R^9$,

$R^8$ einen $C_1$-$C_8$-Alkylrest, einen $C_1$-$C_6$-Halogenalkylrest oder einen gegebenenfalls ein- oder mehrfach durch Halogen, Nitro, Methyl oder Methoxy substituierten Phenylrest und

$R^9$ einen $C_1$-$C_6$-Alkylrest, einen Trifluormethylrest, einen Trichlormethylrest oder einen gegebenenfalls durch Halogen, Nitro oder $C_1$-$C_6$-Alkyl substituierten Phenylrest

bedeuten.

2. Verfahren zur Herstellung von Verbindungen der Formel I, dadurch gekennzeichnet, daß man

A) falls A = COOH und B = Wasserstoff bedeuten, eine Verbindung der allgemeinen Formel II

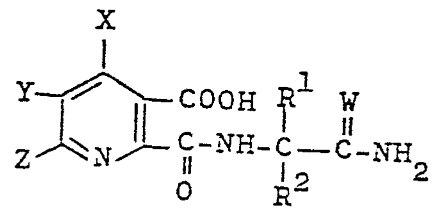

(II) ,

in der $R^1$, $R^2$, W, X, Y und Z die unter der allgemeinen Formel I angegebenen Bedeutungen haben, unter alkalischen Bedingungen cyclisiert,

B) falls A = $COOR^3$, $R^3$ aber nicht für ein Wasserstoffatom steht, und B = Wasserstoff bedeuten, eine Verbindung der allgemeinen Formel III

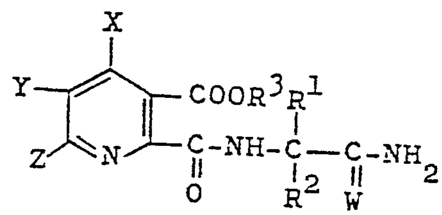

(III) ,

in der $R^1$, $R^2$, $R^3$, W, X, Y und Z die unter der allgemeinen Formel I angegebenen Bedeutungen haben, $R^3$ aber nicht für ein Wasserstoffatom steht, mit einem Gemisch aus Pnosphoroxychlorid und Phosphorpentachlorid umsetzt,

C) falls A = $COOR^3$, $R^3$ aber nicht für ein Wasserstoffatom steht, $CONR^4R^5$ oder $CH_2OH$ und B = Wasserstoff bedeuten, ein Imidazopyrrolopyridin der allgemeinen Formel IV

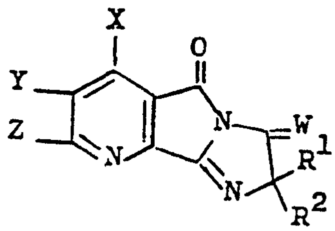

(IV) ,

in der $R^1$, $R^2$, W, X, Y und Z die unter der allgemeinen Formel I angegebenen Bedeutungen haben,

C1) mit einem Alkohol der allgemeinen Formel

$$R^3 - OH,$$

in der $R^3$ die unter der allgemeinen Formel I angegebenen Bedeutungen hat, aber nicht für ein Wasserstoffatom steht, und dem entsprechenden Alkalimetallalkoxid gegebenenfalls in Gegenwart eines Lösungsmittels umsetzt, oder

C2) mit einem Amin der allgemeinen Formel

$$HNR^4R^5,$$

in der $R^4$ und $R^5$ die unter der allgemeinen Formel I angegebenen Bedeutungen haben, gegebenenfalls unter Verwendung eines Lösungsmittels umsetzt, oder

C3) mit einem Redutkionsmittel, wie Natriumborhydrid, zur Reaktion bringt,

D) falls A = COOH bedeutet, eine Verbindung der allgemeinen Formel V

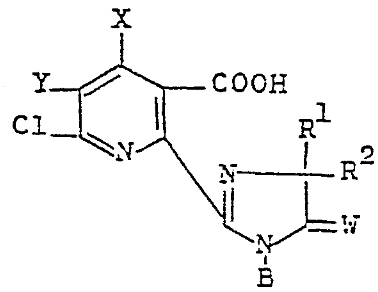

(V) ,

in der B, $R^1$, $R^2$, W, X und Y die unter der allgemeinen Formel I angegebene Bedeutung haben, mit einer Verbindung der allgemeinen Formel

ZH,

wobei Z die unter der allgemeinen Formel I angegebene Bedeutung hat, oder eine Verbindung der allgemeinen Formel XI

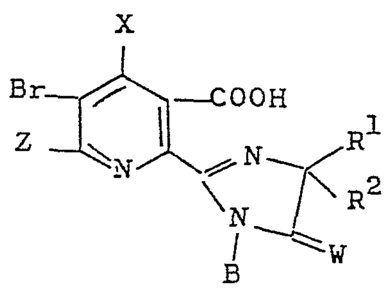

(XI) ,

in der B, $R^1$, $R^2$, W, X und Z die unter der allgemeinen Formel I angegebene Bedeutung haben, mit einer Verbindung der Formel

YH,

wobei Y die unter der allgemeinen Formel I angegebene Bedeutung hat, umsetzt,

E) falls $R^6$ oder $R^7$ im Substituenten Y oder Z nicht Wasserstoff bedeutet, eine Verbindung der allgemeinen Formel I, in der A, B, $R^1$, $R^2$, W, X, Y und Z die unter der allgemeinen Formel I angegebene Bedeutung haben, jedoch Y oder Z eine SH- oder OH-Gruppe darstellen, alkyliert,

F) gewünschtenfalls eine nach den Verfahrensvarianten A) und D) erhaltende Verbindung der allgemeinen Formel I, in der $R^3$ im Substituenten A für Wasserstoff steht, durch Umsetzung mit einem Äquivalent einer Base als Salzbildner in eine Verbindung der allgemeinen Formel I überführt, in der $R^3$ im Substituenten A für ein Kation steht, oder

G) falls B = $COR^8$ oder $SO_2R^9$ bedeutet, eine nach den Verfahrensvarianten B) oder C) erhaltene Verbindung der allgemeinen Formel I, in der A für $COOR^3$ steht, mit einem Acylhalogenid, Acylanhydrid oder Sulfonylhalogenid umsetzt.

3. Mittel mit herbizider Wirkung, gekennzeichnet durch einen Gehalt an mindestens einer Verbindung gemäß Anspruch 1.

4. Mittel gemäß Anspruch 3 in Mischung mit Träger- und/oder Hilfsstoffen.

5. Mittel gemäß Anspruch 3, hergestellt nach einem Verfahren gemäß Anspruch 2.

6. Verwendung der Verbindungen gemäß Anspruch 1 zur Bekämpfung dikotyler und monokotyler Pflanzenarten in Nutzpflanzenkulturen.

Patentansprüche für den Vertragsstaat: AT

1. Mittel mit herbizider Wirkung, gekennzeichnet durch einen Gehalt an mindestens einem 2-(2-Imidazolin-2-yl)-pyridin-3-carbonsäurederivat der allgemeinen Formel I

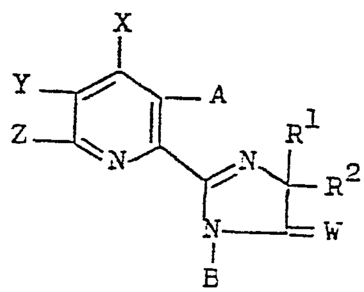

(I) ,

in der

$R^1$ einen $C_1$-$C_4$-Alkylrest,

$R^2$ einen $C_1$-$C_6$-Alkylrest oder einen $C_3$-$C_6$-Cycloalkylrest oder

$R^1$ und $R^2$ gemeinsam mit dem benachbarten Kohlenstoffatom ei- gegebenenfalls durch Methyl substituierten $C_3$-$C_6$-Cycloalkylrest,

W ein Sauerstoff- oder Schwefelatom,

A eine der Gruppen $CH_2OH$, $COOR^3$ oder $CONR^4R^5$,

$R^3$ ein Wasserstoffatom, einen gegebenenfalls durch ein oder mehrere Sauerstoff- oder Schwefelatome unterbrochenen und gegebenenfalls durch $C_1$-$C_3$-Alkoxy, Halogen, Hydroxy, $C_3$-$C_6$-Cycloalkyl, Benzyl, Furyl, Tetrahydrofuryl, Phenyl, Halgenphenyl, $C_1$-$C_4$-Alkylphenyl, $C_1$-$C_4$-Alkoxyphenyl, Nitrophenyl, Cyano, Carboxyl, $C_1$-$C_4$-Alkoxycarbonyl oder $C_1$-$C_4$-Alkylthio substituierten $C_1$-$C_{12}$-Alkylrest, einen gegebenenfalls durch $C_1$-$C_3$-Alkoxy, Phenyl oder Halogen substituierten $C_3$-$C_8$-Alkenylrest, einen gegebenenfalls durch $C_1$-$C_3$-Alkoxy, Phenyl oder Halogen substituierten-$C_3$-$C_8$-Alkinylrest, einen gegebenenfalls durch Methyl substituierten $C_3$-$C_6$-Cycloalkylrest oder ein Kation aus der Gruppe der Alkalimetalle, Erdalkalimetalle, Mangan, Kupfer, Eisen, Zink, Ammonium und organische Ammoniumverbindungen,

$R^4$ und $R^5$ unabhängig voneinander ein Wasserstoffatom, eine Hydroxygruppe oder einen $C_1$-$C_4$-Alkylrest und für den Fall daß $R^4$ ein Wasserstoffatom ist, $R^5$ auch einen Amino-, Dimethylamino-, Acetylamino- oder Anilinorest,

X ein Wasserstoffatom, eine Nitrogruppe, einen $C_1$-$C_3$-Alkylrest, einen $C_1$-$C_3$-Halogenalkylrest oder einen $C_1$-$C_3$-Alkoxyrest und einer der beiden Reste

Y oder Z eine der Gruppen $SR^6$ oder $OR^7$ und der jeweils andere ein Wasserstoffatom, ein Halogenatom, einen $C_1$-$C_4$-Alkylrest, einen $C_1$-$C_4$-Halogenalkylrest, einen $C_1$-$C_4$-Alkoxyrest oder einen $C_1$-$C_4$-Alkylthiorest,

$R^6$ ein Wasserstoffatom, einen Phenylrest, einen Halogenphenylrest, einen $C_1$-$C_3$-Alkylphenylrest, einen Di-$C_1$-$C_3$-alkylphenylrest, einen Nitrophenylrest, einen $C_1$-$C_3$-Alkoxyphenylrest, einen Pyridylrest, einen $C_1$-$C_3$-Alkyl-pyridylrest, einen ein- oder mehrfach durch Sauerstoff unterbrochenen $C_2$-$C_8$-Alkylrest, einen gegebenenfalls durch Sauerstoff, Schwefel, die Imino- oder $C_1$-$C_4$-Alkyliminogruppe unterbrochenen und gegebenenfalls ein- oder mehrfach durch $C_1$-$C_4$-Alkyl, $C_1$-$C_4$-Alkoxy oder Halogen substituierten $C_3$-$C_8$-Cycloalkylrest, einen $C_5$-$C_8$-Cycloalkenylrest oder einen gegebenenfalls ein- oder mehrfach durch Cyano, Halogen, Hydroxy, Mercapto, Amino, $C_1$-$C_6$-Alkoxy, $C_1$-$C_4$-Alkylthio, Carboxyl, Carbamoyl, $C_1$-$C_4$-Alkoxycarbonyl, $C_1$-$C_4$-Alkylthiocarbonyl, Oxo, Thioxo, Di-$C_1$-$C_3$-alkoxy, Hydroxyimino, $C_3$-$C_8$-Cycloalkyl, $C_3$-$C_8$-Cycloalkenyl, Phenyl, Halogenphenyl, $C_1$-$C_3$-Alkylphenyl, Nitrophenyl, $C_1$-$C_3$-Alkoxyphenyl, Furyl, Tetrahydrofuryl, Dioxolanyl, $C_1$-$C_6$-Alkylamino, $C_3$-$C_8$-Cycloalkylamino, Pyridyl, $C_1$-$C_4$-Alkylpyridyl, Pyrrolidinyl, Piperidinyl, N-$C_1$-$C_4$-Alkylpiperidinyl, $C_1$-$C_4$-Alkylcyclopropyl, Di-$C_1$-$C_4$-alkyl-dioxolanyl substituierten $C_1$-$C_{12}$-Alkyl-, $C_3$-$C_{12}$-Alkenyl- oder $C_3$-$C_{12}$-Alkinylrest,

$R^7$ ein Wasserstoffatom, einen gegebenenfalls durch Sauerstoff, Schwefel, die Imino- oder $C_1$-$C_4$-Alkyliminogruppe unterbrochenen und gegebenenfalls ein- oder mehrfach

durch $C_1$-$C_4$-Alkyl, $C_1$-$C_4$-Alkoxy oder Halogen substituierten $C_3$-$C_8$-Cycloalkylrest, einen $C_5$-$C_8$-Cycloalkenylrest oder einen gegebenenfalls ein- oder mehrfach durch Cyano, Hydroxy, Mercapto, Amino, Hydroxy-$C_1$-$C_6$-alkyl, $C_1$-$C_6$-Alkoxy, $C_1$-$C_4$-Alkylthio, Carboxyl, Carbamoyl, Acetyl, $C_1$-$C_4$-Alkoxycarbonyl, $C_1$-$C_4$-Alkylthiocarbonyl, Oxo, Thioxo, Di-$C_1$-$C_3$-alkoxy, Hydroxyimino, $C_3$-$C_8$-Cycloalkyl, Dioxolanyl, $C_5$-$C_8$-Cycloalkenyl, Furyl, Tetrahydrofuryl, $C_1$-$C_6$-Alkylamino, $C_3$-$C_8$-Cycloalkylamino, Di-$C_1$-$C_6$-alkylamino, Pyranyl, Tetrahydropyranyl, Pyridyl, $C_1$-$C_4$-Alkylpyridyl, Pyrrolidinyl, Piperidinyl, N-$C_1$-$C_4$-Alkylpiperidinyl, $C_1$-$C_4$-Alkylcyclopropyl, Di-$C_1$-$C_4$-alkyldioxolanyl substituierten $C_1$-$C_{12}$-Alkyl-, $C_3$-$C_{12}$-Alkenyl- oder $C_3$-$C_{12}$-Alkinylrest,

B ein Wasserstoffatom und für den Fall, daß A = $COOR^3$ darstellt, wobei aber $R^3$ kein Wasserstoffatom bedeutet, auch die Gruppen $COR^8$ oder $SO^2R^9$,

$R^8$ einen $C_1$-$C_8$-Alkylrest, einen $C_1$-$C_6$-Halogenalkylrest oder einen gegebenenfalls ein- oder mehrfach durch Halogen, Nitro, Methyl oder Methoxy substituierten Phenylrest und

$R^9$ einen $C_1$-$C_6$-Alkylrest, einen Trifluormethylrest, einen Trichlormethylrest oder einen gegebenenfalls durch Halogen, Nitro oder $C_1$-$C_6$-Alkyl substituierten Phenylrest

bedeuten, als wirksamen Bestandteil.

2. Verfahren zur Herstellung von Verbindungen der Formel I, dadurch gekennzeichnet, daß man

A) falls A = COOH und B = Wasserstoff bedeuten, eine Verbindung der allgemeinen Formel II

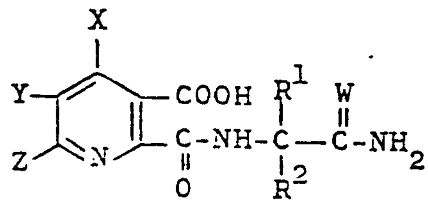

(II) ,

in der $R^1$, $R^2$, W, X, Y und Z die unter der allgemeinen Formel I angegebenen Bedeutungen haben, unter alkalischen Bedingungen cyclisiert,

B) falls A = $COOR^3$, $R^3$ aber nicht für ein Wasserstoffatom steht, und B = Wasserstoff bedeuten, eine Verbindung der allgemeinen Formel III

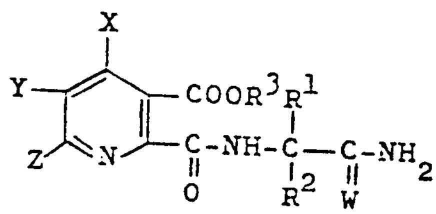

(III) ,

in der $R^1$, $R^2$, $R^3$, W, X, Y und Z die unter der allgemeinen Formel I angegebenen Bedeutungen haben, $R^3$ aber nicht für ein Wasserstoffatom steht, mit einem Gemisch aus Phosphoroxychlorid und Phosphorpentachlorid umsetzt,

C) falls A = $COOR^3$, $R^3$ aber nicht für ein Wasserstoffatom steht, $CONR^4R^5$ oder $CH_2OH$ und B = Wasserstoff bedeuten, ein Imidazopyrrolopyridin der allgemeinen Formel IV

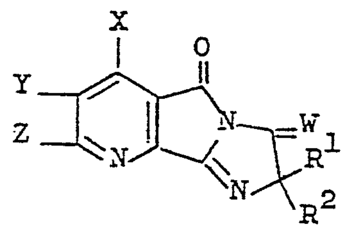

(IV) ,

in der $R^1$, $R^2$, W, X, Y und Z die unter der allgemeinen Formel I angegebenen Bedeutungen haben,

C1) mit einem Alkohol der allgemeinen Formel

$$R^3 - OH,$$

in der $R^3$ die unter der allgemeinen Formel I angegebenen Bedeutungen hat, aber nicht für ein Wasserstoffatom steht, und dem entsprechenden Alkalimetallalkoxid gegebenenfalls in Gegenwart eines Lösungsmittels umsetzt, oder

C2) mit einem Amin der allgemeinen Formel

$$HNR^4R^5,$$

in der $R^4$ und $R^5$ die unter der allgemeinen Formel I angegebenen Bedeutungen haben, gegebenenfalls unter Verwendung eines Lösungsmittels umsetzt, oder

C3) mit einem Redutkionsmittel, wie Natriumborhydrid, zur Reaktion bringt,

D) falls A = COOH bedeutet, eine Verbindung der allgemeinen Formel V

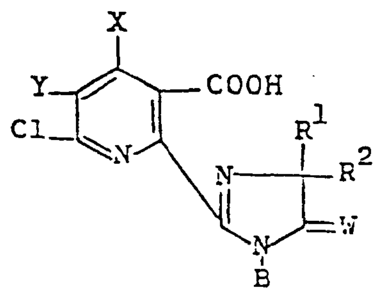

(V),

in der B, $R^1$, $R^2$, W, X und Y die unter der allgemeinen Formel I angegebene Bedeutung haben, mit einer Verbindung der allgemeinen Formel

ZH,

wobei Z die unter der allgemeinen Formel I angegebene Bedeutung hat, oder eine Verbindung der allgemeinen Formel XI

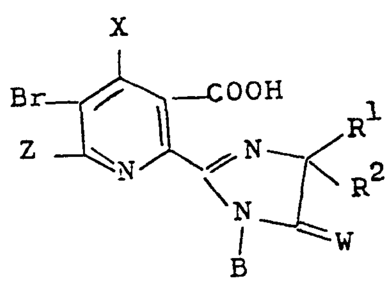

(XI),

in der B, $R^1$, $R^2$, W, X und Z die unter der allgemeinen Formel I angegebene Bedeutung haben, mit einer Verbindung der Formel

YH,

wobei Y die unter der allgemeinen Formel I angegebene Bedeutung hat, umsetzt,

E) falls $R^6$ oder $R^7$ im Substituenten Y oder Z nicht Wasserstoff bedeutet, eine Verbindung der allgemeinen Formel I, in der A, B, $R^1$, $R^2$, W, X, Y und Z die unter der allgemeinen Formel I angegebene Bedeutung haben, jedoch Y oder Z eine SH- oder OH-Gruppe darstellen, alkyliert,

F) gewünschtenfalls eine nach den Verfahrensvarianten A) und D) erhaltende Verbindung der allgemeinen Formel I, in der $R^3$ im Substituenten A für Wasserstoff steht, durch Umsetzung mit einem Äquivalent einer Base als Salzbildner in eine Verbindung der allgemeinen Formel I überführt, in der $R^3$ im Substituenten A für ein Kation steht, oder

G) falls B = $COR^8$ oder $SO_2R^9$ bedeutet, eine nach den Verfahrensvarianten B) oder C) erhaltene Verbindung der allgemeinen Formel I, in der A für $COOR^3$ steht, mit einem Acylhalogenid, Acylanhydrid oder Sulfonylhalogenid umsetzt.

3. Verwendung der Mittel gemäß Anspruch 1 zur Bekämpfung dikotyler und monokotyler Pflanzenarten in Nutzpflanzenkulturen.